(19) Europäisches Patentamt
European Patent Office
Office européen des brevets

(11) Veröffentlichungsnummer: **0 503 438 A2**

## (12) EUROPÄISCHE PATENTANMELDUNG

(21) Anmeldenummer: **92103590.3**

(22) Anmeldetag: **02.03.92**

(51) Int. Cl.5: **C12N 11/00**, C12N 1/04, C02F 3/34, C12P 5/02

(30) Priorität: **15.03.91 DE 4108452**

(43) Veröffentlichungstag der Anmeldung: **16.09.92 Patentblatt 92/38**

(84) Benannte Vertragsstaaten: **AT BE CH DE ES FR GB IT LI NL SE**

(71) Anmelder: **BAYER AG**

**W-5090 Leverkusen 1 Bayerwerk(DE)**

(72) Erfinder: **Pascik, Imre, Dr.**
**Wilmersdorferstrasse 6**
**W-4019 Monheim(DE)**
Erfinder: **Goossens, John, Dipl.-Ing.**
**Hebbelstrasse 5**
**W-5090 Leverkusen(DE)**

(54) **Verfahren zur Herstellung lebendiger, biologisch aktive Mikroorganismen enthaltender Agglomerate.**

(57) Zur Herstellung von lebendigen, aktiven inkorporierten Mikroorganismen oder Enzyme enthaltenden Agglomeraten wird in einen durchrührten Bioreaktor die wässrige Suspension einer adaptierten oder selektiven gezüchteten aktiven, wachstumsfähigen Biomasse in Rein- oder Mischkultur mit einem Biomassentrockensubstanzgehalt von 0,1 g/l bis 15 g/l eingebracht, unter weiterem Rühren auf die Biomassentrockensubstanz bezogen 2 Gew.-% bis 150 Gew.-% eines pulverisierten oder feinkörnigen, festen, adsorbierenden oder ionenaustauschenden Stoffes anorganischer oder organischer Herkunft hinzugefügt und diese Suspension mit einem auf deren gesamte Trockensubstanz bezogenen Anteil von 0,5 Gew.-% bis 30 Gew.-% einer zur Filmbildung und/oder Koagulation fähigen nichtionischen, anionischen oder kationischen Polymerdispersion mit einem Feststoffgehalt von 25 Gew.-% bis 50 Gew.-% 5 bis 20 Minuten bis zur Ausbildung gut sedimentierbarer kompakter Agglomerate intensiv verrührt.

EP 0 503 438 A2

In der biologischen Abwasserbehandlung wird die kontinuierliche oder halbkontinuierliche Zudosierung von pulverisierter Aktivkohle oder Braunkohlenkoks als Trägermaterial oft praktiziert, wobei diese Stoffe nicht nur als Träger für die Biomasse, sondern zwecks Verringerung des Schlammindexes sowie wegen ihrer aktiven Oberfläche auch als Puffer von inhibierenden, adsorbierbaren Abwasserinhaltsstoffen wirken (Korrespondenz Abwasser 2, 129-135 (1987)).

Die Anwendung pulverisierter Kohletypen führt jedoch infolge des Nichtsedimentierens und Auswaschens feinster Kohlepartikel zur Verarmung des Reaktors an Biomasse sowie zur Auflösung von Huminsäuren und damit zu einer Störung des Reaktorablaufs bzw. zu einer Belastung des behandelten Abwassers. Die Zugabe polymerer Flockungshilfsmittel führt zwar zur Ausbildung von Flocken, diese sind aber nicht genug scherstabil, so daß sie bald redispergiert bzw. zerschlagen werden.

Weiterhin muß die ständig zugesetzte Kohle mit dem in der Belebungsstufe gebildeten Überschußschlamm zusätzlich entsorgt werden; damit stellt sie ein weiteres Entsorgungsproblem dar.

In dem Deutschen Patent DE 35 26 184 wird die Herstellung von Agglomeraten aus filmbildenden Polymerdispersionen und feinkörnigen Füllstoffen beschrieben. Dieses Patent befaßt sich insbesondere mit der Herstellung von Trägermaterialien, durch Vermischen der Komponenten in Mischaggregaten wie Schneckentröge, Kneter sowie durch anschließende Koagulation gegebenenfalls unter Anwendung höherer Salzkonzentrationen, pH-Veränderungen bzw. thermischer Behandlung und anschließendem Zerkleinern der derart erhaltenen hohen Trockensubstanzgehalte aufweisenden Granulate.

Diese Methode ist jedoch wegen der für lebendige Zellen extremen Herstellungsbedingungen für eine in situ-Immobilisierung von lebendigen, aktiven Zellen nicht geeignet.

Daher bestand ein Bedarf für eine möglichst einfache technische Bereitstellung von solchen biologisch aktiven, lebendige Zellen enthaltenden Agglomeraten, ohne daß dabei apparative Umbauten an der bestehenden Anlage erforderlich wären. Weitere Zielsetzungen sind höhere Raum-Zeit-Umsätze und höhere Prozeßstabilitäten.

Es wurde erfindungsgemäß gefunden, daß lebendige, immobilisierte Mikroorganismen enthaltende Agglomerate auf relativ einfache Weise "in situ" in dem gerührten Bioreaktor erzeugt werden können, wenn folgende Komponenten miteinander vermischt werden:

I.

Lebendige, suspendierte unter aeroben, fakultativen oder anaeroben Bedingungen wachsende Mikroorganismen,

II.

ein pulverförmiger oder sehr feinkörniger, fester, oberflächenaktiver Stoff wie z.B. pulverisierte Aktivkohle, Braunkohlenschwelkoks, Braunkohle, Ionenaustauscherharz, $Al_2O_3$, $Fe_2O_3$, $Fe_3O_4$, $TiO_2$, $CaCO_3$, Bentonit, Kaolin, Glasmehl usw. sowie

III.

eine zur Filmbildung/Koagulation fähige wäßrige Polymerdispersion.

Dabei kommt es während des Rührvorganges zur Ausbildung von größeren biologisch aktiven Agglomeraten aus den Komponenten, die eine wesentlich höhere Scherfestigkeit, Sinkgeschwindigkeit und Lebensdauer aufweisen als mit Biomasse bewachsene Kohlepartikel, oder mit Hilfe von Polyelektrolyten geflockte biomassenhaltige Kohlepartikel. Unter "Bioreaktor" wird im Rahmen dieser Erfindung ein Reaktor zur Züchtung und Kultivierung von Mikroorganismen einschließlich biologischer Kläranlagen zur Abwasserreinigung (aerob oder anaerob) verstanden. Gegenstand der Erfindung ist also ein Verfahren zur "in situ" Herstellung von lebendigen, aktiven, inkorporierte Mikroorganismen enthaltende Agglomeraten. Dieses Verfahren ist erfindungsgemäß dadurch charakterisiert, daß man

I.

in einen durchrührten Bioreaktor die wäßrige Suspension einer adaptierten oder selektiven gezüchteten aktiven, wachstumsfähigen Biomasse in Rein- oder Mischkultur mit einem Biomassen-Trockensubstanzgehalt von

0,1 g/l bis 15 g/l, bevorzugt

0,15 g/l bis 12 g/l, besonders bevorzugt

0,2 bis 10 g/l

einbringt,

II.

unter weiterem Rühren auf die Biomassentrockensubstanz bezogen

2 Gew.-% bis 150 Gew.-%, bevorzugt

5 Gew.-% bis 120 Gew.-%, besonders bevorzugt

10 Gew.-% bis 100 Gew.-%

eines pulverisierten oder feinkörnigen, festen, adsorbierenden oder ionenaustauschenden Stoffes anorganischer oder organischer Herkunft hinzufügt

und diese Suspension mit einem auf deren gesamte Trockensubstanz bezogenen Anteil von

III.

0,5 Gew.-% bis 30 Gew.-%, bevorzugt

1 Gew.-% bis 25 Gew.-%, und besonders bevorzugt

1,5 Gew.-% bis 20 Gew.-%

einer zur Filmbildung und/oder Koagulation fähigen nichtionischen, anionischen oder kationischen Polymerdispersion mit einem Feststoffgehalt von 25 Gew.-% bis 50 Gew.-% 5 bis 20 Minuten bis zur Ausbildung gut sedimentierbarer kompakter Agglomerate intensiv verrührt.

Bei der Polymerdispersion handelt es sich um wäßrige Dispersionen von durch Polymerisation, Polykondensation oder Polyaddition hergestellten Polymeren, die bevorzugt durch in das Polymergerüst eingebauten anionischen, kationischen oder nichtionischen internen Emulgatoren dispergiert werden.

Besonders gut geeignet sind Dispersionen von Polymerisaten oder Copolymerisaten, hergestellt aus olefinisch ungesättigten Monomeren wie Acrylnitril, Styrol, Methacrylnitril, Acrylsäure, Methacrylsäure, Methylmethacrylat, Allylmethacrylat, Hydroxyalkylacrylat, N,N-Dimethylaminoethylmethacrylat, Acrylamid, Ethan, Propan, Vinylchlorid, Vinylacetat, 1,3-Butadien, 2-Methylbutadien-(1,3), 2,3-Dimethylbutadien-(1,3), 2-Chlorbutadien-(1,3) und p-Divinylbenzol.

Zur Herstellung der erfindungsgemäßen Agglomerate wird in den die suspendierte Biomasse I enthaltenden Bioreaktor zunächst die Komponente II (Additiv) und nach deren guter Verteilung über das gesamte Flüssigkeitsvolumen, unter intensivem Rühren auch die Komponente III (Dispersion) zugegeben, wobei das intensive Rühren noch etwa 15 bis 20 Minuten bis zur vollständigen Bildung gut sedimentierbarer Agglomerate fortgesetzt wird.

Das erfindungsgemäße Verfahren ist besonders in solchen Fällen vorteilhaft, wenn es sich um die Agglomerierung von Stämmen mit geringerem Flockenbildungsvermögen bzw. langsam sedimentierende Flocken bildende Stämme handelt, wie es z.B. die nitrifizierenden Bakterien Nitrosomonas und Nitrobacter sind, sowie andere für die Mineralisierung von langsam abbaubaren Organica fähigen Stämme wie Pseudomonas, Flavobacterium, Rhodococcus, u.a..

Das erfindungsgemäße Verfahren ist weiterhin auch für die Agglomerierung von unter fakultativen und anaeroben Bedingungen wachsenden Kulturen wie beispielsweise für die acidogenen und methanogenen Mikroorganismen Methanosarcina, Methanothrix u.a. geeignet.

Das erfindungsgemäße Agglomerierungsverfahren weist gegenüber den verschiedenen praktizierten Immobilisierungsverfahren mehrere Vorteile auf. Es läßt sich ganz einfach unter sukzessiver Beimischung von nicht toxischen Komponenten im Bioreaktor selbst, unter ganz üblichen Reaktionsbedingungen durchführen, d.h. ohne drastische pH-Änderung, gezielte Temperaturerhöhung, Erhöhung der Ionenstärke durch Salzzugabe, sowie ohne Zugabe toxischer Vernetzungs- bzw. Aushärtungsmittel.

Das "Produkt", d.h. die Agglomerate, verbleiben im Reaktor und werden nicht weiteren für die Herstellung von anderen Trägern üblichen zusätzlichen Verfahrensschritten wie Separieren, Trocknen und Zerkleinerung unterworfen und können direkt weiter verwendet werden.

Weiterhin sind für die Umwälzung, Belüftung und Rückhaltung der Agglomerate keine zusätzlichen Vorrichtungen bzw. Ein- und Umbauarbeiten erforderlich.

Die Anwendung des erfindungsgemäßen Agglomerierungsverfahrens bringt auch mehrere prozeßtechnische Vorteile mit sich, wie z.B. die Verringerung des Schlammindexes, verbesserte Fluidisierbarkeit, erhöhte Prozeßstabilität und höhere Raum-Zeit-Ausbeuten, was in der Praxis eine stabile, problemlose Prozeßführung ermöglicht.

Da die Agglomerate schneller sedimentieren als die suspendierten Schlammflocken gelangen sie in geringerem Maße in die nachgeschalteten Klärer, was für die Praxis eine erhöhte Verweilzeit der Biomasse im Reaktor bedeutet. Dies ist besonders bei Prozessen mit langsam wachsender Biomasse von entscheidendem Vorteil.

Die erfindungsgemäßen Agglomerate weisen infolge der größeren Adhäsion der Komponenten gegenüber mit linearen Flockungshilfsmitteln auf der Basis von Polyacrylamid geflockten Biomassen-/Kohlenflocken eine erhöhte Dimensionsstabilität auf, was ebenfalls zur höheren Prozeßstabilität führt. Die Verbesserung der Prozeßstabilität bei der biologischen Abwasserreinigung kommt vor allem zum Tragen, wenn sich die Zusammensetzung des Abwassers ändert oder Belastungsschwankungen auftreten. In solchen Fällen kann durch eine problemorientierte (hinsichtlich der zum Einsatz gelangenden Mikroorganismenstämme), vorübergehende und automatisch gesteuerte Anwendung des erfindungsgemäßen Verfahrens Abhilfe geschaffen werden.

Latextypen

Als Komponente III können folgende wäßrige Polymerdispersionen verwendet werden:

Latex A: Anionisches Polymerisat, 40 %ige Dispersion aus Butadien, Acrylnitril und Methacrylsäure im Gewichtsverhältnis 62:34:4

Latex B: Anionisches Polymerisat aus Butadien, Styrol und Acrylsäure im Gewichtsverhältnis 41:56:3

Latex C: Polymerisat aus Butadien und Styrol im Gewichtsverhältnis 68:32

Latex D: Kationisches Polymerisat aus Butadien, Styrol und kationisches Comonomer im Gewichtsverhältnis 38:58:4

Die Beispiele 1 bis 5 dienen zur Erläuterung der Erfindung. Während in Beispiel 1 die Herstellung der Agglomerate und die dabei erreichten höheren Absetzgeschwindigkeiten ohne biologische Anwendung erläutert wird, stehen bei den Beispielen 2 bis 5 der Darstellung die erhöhte biologische Aktivität und Prozeßstabilität durch die Agglomerierung im Vordergrund.

Beispiel 1

In 5 mit Magnetrührer ausgerüstete 2000 ml fassende Bechergläser werden je 1000 ml, 4 g/l nitrifizierende Mikroorganismen enthaltende Biomassensuspension (Komponente I) eingefüllt. Unter intensivem Rühren werden jeweils 1 g der Komponente II sowie danach 0,1 g Polymerisatdispersion (Komponente III) zugefügt. Nach ca. 15 Minuten ist die Agglomerierung abgeschlossen. In einem 1000 ml fassenden graduierten Meßzylinder werden die Absetzgeschwindigkeiten und Schlammvolumina der Agglomerate bestimmt. Die Ergebnisse sind in der nachfolgenden Tabelle dargestellt:

Beispiel 2

(Nitrifikation eines ammoniumreichen Abwassers)

EP 0 503 438 A2

| Versuch | | Komponente II | Komponente III | Schlammvolumina (ml/l) nach | | | |
|---|---|---|---|---|---|---|---|
| | | | | 4 Min. | 8 Min. | 16 Min. | 30 Min. |
| | a) | nicht immobilisierte Biomasse | | 740 | 540 | 430 | |
| A | b) | Braunkohlen-schwelkoks | Latex A | 610 | 415 | 335 | |
| | a) | nicht immobilisierte Biomasse | | 700 | 380 | 280 | |
| B | b) | Braunkohlen-schwelkoks | Latex B | 480 | 310 | 240 | |
| | a) | nicht immobilisierte Biomasse | | - | 485 | 465 | |
| C | b) | Braunkohlen-schwelkoks | Latex B | - | 465 | 430 | |
| | c) | CaCO$_3$-Pulver | Latex D | - | 410 | 315 | |

Zwei parallel betriebene, aus 7 l fassenden belüfteten Bioreaktoren sowie nachgeachaltetem 3 l fassenden Nachklärer und Schlammrücklauf bestehenden Aerobanlagen, werden mit einer 4 g/l nitrifizierenden Biomassentrockensubstanz enthaltenden Belebtschlammsuspension angeimpft und kontinuierlich mit einem

1400 bis 2000 mg/l $NH_4$-N,
500 bis 1000 mg/l Ethanol,
300 mg/l Phenol und
500 mg/l 2-Naphthalinsulfonsäure

enthaltenden synthetischen Abwasser beschickt, um die Nitrifikation dieses Abwassers mit suspendierten (Reaktor A) und immobilisierten (Reaktor B) Mikroorganismen vergleichend untersuchen zu können.

In Reaktor B werden nach Zugabe der Biomasse 9 g/l neutralisiertes Braunkohlenschwelkokspulver ("Braunkohlenfeinstkoks": Produktbezeichnung der Rheinischen Braunkohlenwerke AG, Köln, Deutschland) sowie unter intensiver Luftumwälzung 0,5 g/l 40 % Trockensubstanz enthaltenden Latex A zugegeben.

Nach ca. 10 Minuten wird die leicht schäumende, milchig trübe wäßrige Phase klarer, wobei die Agglomeratpartikel frei erkennbar werden.

Die beiden parallel betriebenen Anlagen werden in den ersten Tagen bei einer $NH_4$-N-Raumbelastung von 0.3 - 0.4 g $NH_4$-N-Raumbelastung von 0,3 bis 0,4 g $NH_4$-N/l · Tag betrieben, danach wird die Belastung sukzessive erhöht. Die Ergebnisse des 83 Tage kontinuierlich laufenden Versuchs werden in der nachfolgenden Tabelle dargestellt:

Wie die erreichten Ergebnisse zeigen, kann man durch das Agglomerieren (Immobilisierung) der Mikroorganismen bei erheblich höheren $NH_4$-Raumbelastungen eine weitgehende Nitrifikation bei einer hohen Prozeßstabilität erreichen.

| Versuchs-tag | Reaktor | N-Raumbelastung g $NH_4$-N/l · Tag | $NH_4$-N-Konzentration (mg/l) Zulauf | Ablauf | % Elimination |
|---|---|---|---|---|---|
| 10 | A | 0,8 | 820 | 5 | 99,4 |
|    | B | 1,2 | 820 | 5 | 99,5 |
| 31 | A | 1,38 | 1420 | 160 | 88,7 |
|    | B | 1,42 | 1420 | 5 | 99,6 |
| 40 | A | 1,82 | 1730 | 240 | 86,1 |
|    | B | 1,82 | 1730 | 5 | 99,7 |
| 47 | A | 2,1 | 2120 | 630 | 70,3 |
|    | B | 2,15 | 2120 | 10 | 99,5 |

## Beispiel 3

Zwei 7 l fassende, parallel betriebene im Beispiel 1 beschriebenen Aerobanlagen werden gemäß Beispiel 1 mit je 4 g/l suspendierter nitrifizierender Biomasse angeimpft. Die im Reaktor B befindliche Biomasse wird unter Zusatz von 1 g/l Braunkohlenschwelkoks und 0,1 g/l 40 % Trockensubstanz enthaltenden Latex A immobilisiert.

Als Substrat dient eine 80 bis 120 mg/l $NH_4$-N enthaltende Mischung aus 60 bis 80 Vol.-% neutralisiertes Chemiemischabwasser, sowie 20 bis 40 % Kommunalabwasser.

7

Die beiden Reaktoren wurden nahezu parallel betrieben, d.h. die hydraulische Verweilzeit und $NH_4$-N-Raumbelastung wurden in beiden Reaktoren etwa gleich geschaltet. Die Ergebnisse des Versuches werden in der nachfolgenden Tabelle dargestellt:

| Versuchs-tag | Reaktor | N-Raumbelastung g $NH_4$-N/l · Tag | $NH_4$-N-Konzentration (mg/l) Zulauf | Ablauf | % Elimination |
|---|---|---|---|---|---|
| 11  14.04. | A | 0,11 | 90 | 97 | - |
|  | B | 0,11 | 90 | 58 | 35,6 |
| 48  18.06. | A | 0,12 | 89 | 62 | 30,3 |
|  | B | 0,12 | 89 | 9 | 89,9 |
| 187  09.10. | A | 0,13 | 104 | 65 | 37,5 |
|  | B | 0,14 | 104 | 3 | 97,1 |

Während sich im Reaktor A die Nitrifikation nie richtig "etablieren" konnte, verlief sie im Reaktor B nach etwa 3 Wochen dauernder Adaptionsphase sehr stabil und mit hoher Umsatzrate.

Beispiel 4 (Nitrifikation von Kommunalabwasser)

Zwei 25 l fassende, parallel betriebene Aerobanlagen werden mit je 4 g/l nitrifizierender Biomasse angeimpft. Die im Reaktor B befindliche Biomasse wird unter Zusatz von 1 g/l Braunkohlenschwelkoks und 0,1 g/l Latex A immobilisiert. Als Substrat dient mechanisch vorgeklärtes Kommunalabwasser, dem zusätzlich zum natürlichen $NH_4^+$-N-Gehalt noch 50 mg/l $NH_4^+$-N zugefügt wurden. Die Ergebnisse des Nitrifikationsversuches werden in der nachfolgenden Tabelle dargestellt:

| Versuchs-tag | Reaktor | N-Raumbelastung g $NH_4$-N/l · Tag | $NH_4$-N-Konzentration (mg/l) | | % Elimination |
|---|---|---|---|---|---|
| | | | Zulauf | Ablauf | |
| 1 | A | 0,40 | 93 | 11 | 88,5 |
| | B | 0,41 | 93 | 10,2 | 89,0 |
| 7 | A | 0,36 | 82 | 15,0 | 81,3 |
| | B | 0,38 | 82 | 5,4 | 93,4 |
| 17 | A | 0,42 | 95 | 64 | 32,6 |
| | B | 0,42 | 95 | 8,5 | 91,1 |
| 27 | A | 0,50 | 120 | 48 | 56,4 |
| | B | 0,50 | 120 | 18 | 85,0 |

Beispiel 5

EP 0 503 438 A2

Zwei parallel betriebene aus 8,7 l fassenden, thermostatisierten, gerührten Methanreaktor, Siphon sowie Gasbürette bestehende Anaerobanlagen werden mit je 10 g/l Biomassentrockensubstanz enthaltendem Anaerobschlamm befüllt. In Reaktor B werden 2 g/l Quarzsand sowie unter intensivem Rühren 0,2 g/l Latex D zugegeben. Nach 15 Minuten Rühren wird die Anlage kontinuierlich mit einem Mischabwasser (Brüdenkondensate + Alkaliextrakt der Chlorbleiche) einer Sulfitzellstoffabrik beschickt. Die Ergebnisse des kontinuierlichen Versuchs der anaeroben Behandlung werden in der nachfolgenden Tabelle dargestellt:

| Versuchs-tag | Reaktor | CSB-Raumbelastung g CSB/l · Tag | CSB (mg/l) Zulauf | CSB (mg/l) Ablauf | % Elimination |
|---|---|---|---|---|---|
| 5 | A | 0,95 | 5330 | 1760 | 67,0 |
| | B | 0,95 | 5330 | 1390 | 73,9 |
| 10 | A | 1,12 | 5510 | 2330 | 57,7 |
| | B | | 5510 | 1780 | 67,7 |
| 23 | A | 1,23 | 5330 | 1961 | 63,2 |
| | B | 1,23 | 5330 | 1270 | 76,2 |
| 58 | A | 1,6 | 4890 | 2820 | 42,3 |
| | B | 1,6 | 4890 | 1700 | 62,2 |

**Patentansprüche**

1. Verfahren zur in situ Herstellung von lebendigen, aktiven inkorporierten Mikroorganismen (und/oder Enzyme) enthaltenden Agglomeraten, dadurch gekennzeichnet, daß man

I.

in einen durchrührten Bioreaktor die wäßrige Suspension einer adaptierten oder selektiven gezüchteten aktiven, wachstumsfähigen Biomasse in Rein- oder Mischkultur mit einem Biomassen-Trockensubstanzgehalt von

0,1 g/l bis 15 g/l, bevorzugt
0,15 g/l bis 12 g/l, besonders bevorzugt
0,2 g/l bis 10 g/l

einbringt,

II.

unter weiterem Rühren auf die Biomassentrockensubstanz bezogen

2 Gew.-% bis 150 Gew.-%, bevorzugt
5 Gew.-% bis 120 Gew.-%, besonders bevorzugt
10 Gew.-% bis 100 Gew.-%

eines pulverisierten oder feinkörnigen, festen, adsorbierenden oder ionenaustauschenden Stoffes anorganischer oder organischer Herkunft hinzufügt

und diese Suspension mit einem auf deren gesamte Trockensubstanz bezogenen Anteil von

III.

0,5 Gew.-% bis 30 Gew.-%, bevorzugt
1 Gew.-% bis 25 Gew.-%, und besonders bevorzugt
1,5 Gew.-% bis 20 Gew.-%

einer zur Filmbildung und/oder Koagulation fähigen nichtionischen, anionischen oder kationischen Polymerdispersion mit einem Feststoffgehalt von 25 Gew.-% bis 50 Gew.-% 5 bis 20 Minuten bis zur Ausbildung gut sedimentierbarer kompakter Agglomerate intensiv verrührt.

2. Verfahren nach Anspruch 1, dadurch gekennzeichnet, daß als Mikroorganismen Nitrosomonas, Nitrobacter, Pseudomonas für die Nitrifiketion von Ammonium verwendet werden.

3. Verfahren nach Anspruch 1, dadurch gekennzeichnet, daß als Mikroorganismen Pseudomonas, Flavabacterium, Bacillus, Rhodococcus usw. sowie deren Mischungen für die Mineralisierung biologisch langsam abbaubarer organischer Substanzen unter fakultativen oder aeroben Bedingungen verwendet werden.

4. Verfahren nach Anspruch 1, dadurch gekenzeichnet, daß als Mikroorganismen Methanothrix, Methanosarcina und andere methanogene Organismen oder deren Mischungen für die Methanisierung geeigneter organischer Substrate verwendet werden.

5. Verfahren gemäß Anspruch 1 bis 4, dadurch gekennzeichnet, daß man als wäßrige Polymerdispersionen zur Filmbildung und Koagulation fähige Polymerisatdispersionen auf der Basis von olefinisch ungesättigten Monomeren verwendet.

6. Verwendung der gemäß Anspruch 1 bis 5 erhaltenen Agglomerate bei der biologischen Abwasserreinigung und bei Biokonversionsprozessen.